# EUROPÄISCHE PATENTSCHRIFT

(11) **EP 0 392 233 B1**
(45) Veröffentlichungstag und Bekanntmachung des Hinweises auf die Patenterteilung: **21.09.1994**
(21) Anmeldenummer: 90105519.4
(22) Anmeldetag: 23.03.1990
(51) Int. Cl.: C07D 273/00, A61K 31/41

(54) **Substituierte 1.2.3.4-Oxatriazolium-5-olate, Verfahren zu ihrer Herstellung und ihre Verwendung**
Substituted 1,2,3,4,-Oxatriazolium-5-olates, method for their preparation and their use
1,2,3,4,-Oxatriazolium-5-olates substitués, procédé pour leur préparation et leur utilisation

(30) Priorität: 10.04.1989 DE 3911668
(43) Veröffentlichungstag der Anmeldung: 17.10.1990
(73) Patentinhaber: CASSELLA Aktiengesellschaft, D-60386 Frankfurt (DE)
(72) Erfinder: Schönafinger, Karl, Dr., D-8755 Alzenau (DE); Bohn, Helmut, Dr., D-6369 Schöneck 1 (DE); Beyerle, Rudi, Dr., D-6000 Frankfurt am Main 60 (DE); Just, Melitta, Dr., D-6369 Nidderau 2 (DE)
(74) Vertreter: Muley, Ralf, Dr.

(56) Entgegenhaltungen:
- GB-A- 1 065 684
- Chemical Abstracts, Band 72, Nr. 17, 27. April 1970, Seite 389, Zusammenfassung-Nr. 90382q, Columbus, Ohio, US; T.L. Thomas et al.: "Synthesis and activity of some 3-substituted 1,2,3,4-pseudooxatriazol-5-ones and their precursors and related compounds."
- Chemical Abstracts, Band 66, Nr. 5, 30. Januar 1967, Seite 1705, Zusammenfassung-Nr. 17798b, Columbus, Ohio, US; L.B. Kier et al.: "Mesoionic -oxatriazoles as hypotensive agents"
- Chemical Abstracts, Band 65, Nr. 3, 2. August 1966, Seite 4442, Zusammenfassung-Nr. 4442e, Columbus, Ohio, US; L.B. Kier et al.: "A new class of hypotensive agents"

## Beschreibung

Die Erfindung betrifft neue in 3-Stellung substituierte 1.2.3.4-Oxatriazolium-5-olate der allgemeinen Formel I 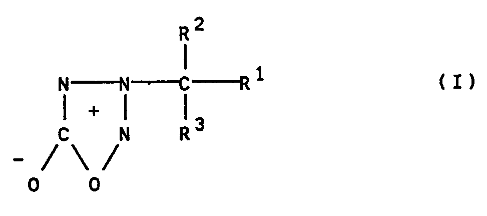
worin
- R¹: (C₂ bis C₄)Alkyl oder -CONR⁴R⁵,
- R², R³: unabhängig voneinander (C₁ bis C₄)Alkyl,
- R⁴: Wasserstoff, (C₁ bis C₄)Alkyl und
- R⁵: (C₁ bis C₄)Alkyl bedeuten, oder
- R⁴ und R⁵: zusammen mit dem Stickstoffatom, an das sie gebunden sind, einen 5-, 6- oder 7-gliedrigen heterocyclischen Ring bilden, der auch noch ein oder mehrere Heteroatome aus der Reihe N, O oder S enthalten kann.

Die Erfindung betrifft auch Verfahren zur Herstellung der Verbindungen der Formel I und ihre Verwendung.

Für die Verbindungen der Formel I können auch die Formel Ia 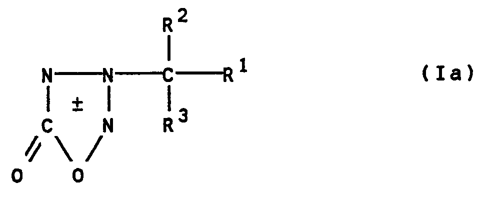
und mesomere Grenzstrukturen, z.B. die der Formeln Ib bis If, angegeben werden: 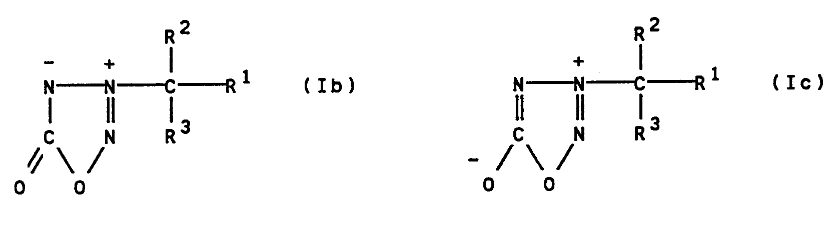 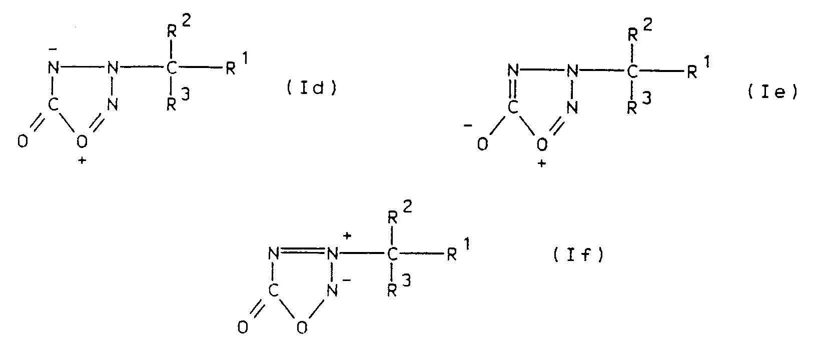
Die für R¹, R², R³, R⁴ und R⁵ stehenden Alkylreste können geradkettig oder verzweigt sein.

Beispiele für (C₂ bis C₄)Alkylreste, die für R¹, R², R³, R⁴ und R⁵ stehen können, sind Ethyl, Propyl, i-Propyl, Butyl, i-Butyl, sec-Butyl, tert-Butyl. Für R², R³, R⁴ und R⁵ ist darüberhinaus auch noch Methyl möglich. R² und R³ können gleich oder verschieden sein und bedeuten vorzugsweise Ethyl oder Methyl und sind vorzugsweise gleich. Ganz besonders bevorzugt bedeuten R² und R³ beide Methyl.

Für R¹ sind die (C₂ bis C₄)Alkylreste bevorzugt. Ganz besonders bevorzugt ist für R¹ der Ethylrest.

Die für R², R³, R⁴ und/oder R⁵ stehenden Alkylreste sind vorzugsweise unverzweigt. Beispiele für die für R¹ stehenden Reste -CONR⁴R⁵ sind: Methylamino-carbonyl, Ethylamino-carbonyl, i-Propylamino-carbonyl, i-Butylamino-carbonyl, N,N-Diethylamino-carbonyl, N,N-Dimethylamino-carbonyl, N,N-Di-n-propylamino-carbonyl, N,N-Di-n-butylamino-carbonyl.

R⁴ und R⁵ können zusammen mit dem Stickstoffatom, an das sie gebunden sind, einen heterocyclischen Ring, insbesondere einen 5-, 6- oder 7-gliedrigen heterocyclischen Ring bilden, der auch noch ein oder mehrere Heteroatome aus der Reihe N, O oder S enthalten kann. Beispielsweise können R⁴ und R⁵ zusammen mit dem Stickstoffatom, an das sie gebunden sind, einen Pyrrolidin-, Piperidin-, Piperazin- oder Morpholinrest bilden.

Die Verbindungen der allgemeinen Formel I lassen sich durch Cyclisierung von Nitrosoverbindungen der allgemeinen Formel II 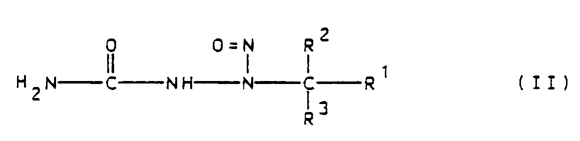
worin R¹, R² und R³ die bereits genannten Bedeutungen besitzen, herstellen.

Die Cyclisierung der Verbindungen der Formel II wird in einem Lösungsmittel durch Erhitzen auf 30°C bis zum Siedepunkt des Lösungsmittels, insbesondere 30°C bis 100°C, vorzugszweise 50 bis 80°C, durchgeführt und kann durch Zusatz einer Säure beschleunigt werden.

Geeignete Lösungsmittel sind z.B.: Wasser; Alkohole, insbesondere solche mit 1 bis 6 C-Atomen, wie z.B. Methanol, Ethanol, i- und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methylethylether, Di-n-propylether, Di-isopropylether, Methyl-n-butylether, Ethylpropylether, Dibutylether, Tetrahydrofuran, 1,4-Dioxan, 1,2-Dimethoxyethan, Bis-β-methoxyethylether; Polyether, wie z.B. Polyethylenglykole mit einem Molekulargewicht bis ca. 600; Glykole und teilweise veretherte Glykole, wie z.B. Ethylenglykol, Propylenglykol, Trimethylenglykol, Ethylenglykol-monomethylether, Ethylenglykol-monoethylether, Diethylenglykolmonoethylether; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Benzophenon, Acetophenon; aliphatische Kohlenwasserstoffe, wie z.B. niedrig- und hochsiedende Petrolether; aromatische Kohlenwasserstoffe, wie z.B. Benzol, Toluol, o-, m- und p-Xylol, halogenierte aliphatische oder aromatische Kohlenwasserstoffe, wie z.B. Methylenchlorid, Chloroform, Tetrachlorkohlenstoff, Ethylenchlorid, Chlorbenzol, Dichlorbenzol; Nitrile, wie z.B. Acetonitril; Amide, wie z.B. Dimethylformamid, N-methyl-pyrrolidon; Sulfoxide, wie z.B. Dimethyl-sulfoxid.

Die Cyclisierung kann auch in einem Gemisch verschiedener Lösungsmittel durchgeführt werden.

Geeignete Säuren zur Beschleunigung der Cyclisierung sind organische oder anorganische Säuren. Geeignete organische Säuren sind z.B. unsubstituierte oder substituierte aliphatische oder aromatische Carbonsäuren, wie z.B. Ameisensäure, Essigsäure, Propionsäure, Buttersäure; ferner Fluoressigsäure, Difluoressigsäure, Trifluoressigsäure, Glykolsäure, Chloressigsäure, Dichloressigsäure, Tri-chloressigsäure, α-Chlorpropionsäure, β-Chlorpropionsäure, Benzoesäure, o-, m- oder p-Toluylsäure, o-, m- oder p-Chlorbenzoesäure, o-, m- oder p-Nitrobenzoesäure.

Geeignete anorganische Säuren sind z.B. Schwefelsäure; Halogenwasserstoffsäuren, insbesondere Salzsäure; Phosphorsäure. Ferner sind z.B. Sulfonsäuren geeignet, wie z.B. Benzolsulfonsäure, p-Toluolsulfonsäure oder Methansulfonsäure.

Durch Zusatz einer Säure kann die Cyclisierung der Verbindungen der Formel II unter Umständen so beschleunigt werden, daß bei der Cyclisierung keine Erwärmung auf höhere Temperaturen mehr erforderlich ist, weil die Cyclisierung bereits bei Raumtemperatur oder niederer Temperatur abläuft.

Bei Zusatz einer Säure kann daher die Cyclisierung in einem Lösungsmittel bei Temperaturen von 5°C bis zum Siedepunkt des Lösungsmittels, vorzugsweise bei Temperaturen von Raumtemperatur bis 100°C, ganz besonders bevorzugt von Raumtemperatur bis zu 80°C, durchgeführt werden.

Die zur Beschleunigung der Cyclisierung benutzten Säuren können gegebenenfalls auch als Lösungsmittel dienen, so z.B. Essigsäure oder Salzsäure.

Die Nitrosoverbindungen der Formel II lassen sich durch Nitrosierung von Semicarbaziden der Formel III mit salpetriger Säure in an sich bekannter Weise herstellen: 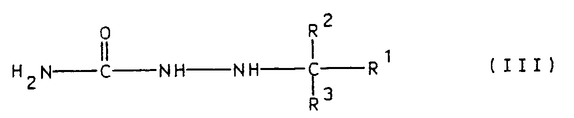
Die Semicarbazide der Formel III lassen sich aus den entsprechenden Hydrazinen der allgemeinen Formel IV 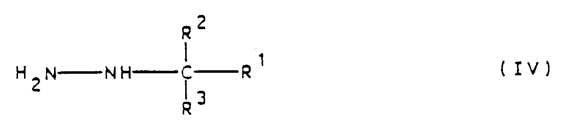
in an sich bekannter Weise, z.B. durch Umsetzung mit Cyanaten gewinnen. Die Hydrazine der Formel IV können durch Monoalkylierung von Hydrazin oder Hydrazinhydrat mit Alkylierungsmitteln der Formel V 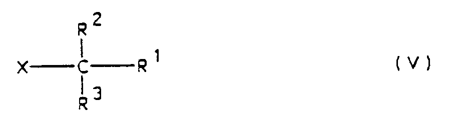
nach an sich bekannten Methoden erhalten werden, wobei X ein Halogen, z.B. Brom, bedeutet. In den Formeln III bis V besitzen R¹, R² und R³ die bereits eingangs genannten Bedeutungen. Die Verbindungen der Formel V sind bekannt oder lassen sich nach bekannten Verfahren darstellen.

Den erfindungsgemäßen Verbindungen ähnliche Verbindungen sind in BP 1 065 684 z.T. als hypotensive und CNS-stimulierende Verbindungen beschrieben.

Die erfindungsgemäßen Verbindungen der Formel I besitzen wertvolle pharmakologische Eigenschaften. Überraschenderweise wurde bei den erfindungsgemäßen Verbindungen der Formel I gefunden, daß sie antianginöse Wirkung entfalten und daß sie bei Angina pectoris eine Entlastung des Herzens bewirken. Außerdem entfalten diese Verbindungen antithrombotische Wirkungen am Tiermodell.

Die erfindungsgemäßen Verbindungen der Formel I sind daher zur Herstellung von Arzneimitteln mit antianginöser oder antithrombotischer Wirkung geeignet.

Die Verbindungen der Formel I können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I neben einem oder mehreren üblichen pharmazeutisch einwandfreien Träger- oder Verdünnungs- und gegebenenfalls einem oder mehreren Zusatzstoffen enthalten.

Die Heilmittel können oral, z.B. in Form von Tabletten, Filmtabletten, Dragees, Hart- und Weichgelatinekapseln, Mikrokapseln, Granulaten, Pulvern, Pellets, Lösungen, Sirupen, Emulsionen, Suspensionen, Aerosolen, Schäumen, Pillen oder Pastillen verabreicht werden. Die Verabreichung kann aber auch rektal, z.B. in Form von Suppositorien, oder parenteral, z.B. in Form von Injektionslösungen, oder perkutan, z.B. in Form von Salben, Cremes, Gelen, Pasten, Aerosolen, Schäumen, Pudern oder Tinkturen, erfolgen.

Die pharmazeutischen Präparate können in an sich bekannter Weise unter Verwendung pharmazeutisch inerter anorganischer oder organischer Hilfs-, Träger-, Füll- oder Verdünnungsstoffe hergestellt werden. Für die Herstellung von Pillen, Tabletten, Filmtabletten, Dragees und die Pellet- oder Granulatfüllungen von Hartgelatinekapseln kann man z.B. Calciumphosphate, Lactose, Sorbitol, Mannitol, Stärken, präparierte Stärken, chemisch modifizierte Stärken, Stärkehydrolysate, Cellulose, Cellulosederivate, synthetische Polymere, Talk etc. verwenden. Träger- oder Verdünnungsstoffe für Weichgelatinekapseln und Suppositorien sind z.B. Fette, Wachse, halbfeste und flüssige Polyole, natürliche oder gehärtete Öle etc. Als Träger- oder Verdünnungsstoffe für die Herstellung von Lösungen und Sirupen eignen sich z.B. Wasser, Polyole, Lösungen von Saccharose, Invertzucker, Glukose etc. Als Trägerstoffe für die Herstellung von Injektionslösungen eignen sich z.B. Wasser, Alkohole, Glyzerol, Polyole oder pflanzliche Öle. Als Träger- oder Verdünnungsstoffe für Salben, Cremes und Pasten eignen sich z.B. Naturvaseline, Kunstvaseline, dick- und dünnflüssige Paraffine, Fette, natürliche oder gehärtete pflanzliche und tierische Öle, Neutralöle, Wachse, Wachsalkohole, Polyethylenglykole, Polyacrylsäure, Silicongele etc.

Die pharmazeutischen Präparate können in an sich bekannter Weise neben den Wirk- und Verdünnungs-, Füll- oder Trägerstoffen auch noch einen oder mehrere Zusatzstoffe oder Hilfsmittel, wie z.B. Spreng-, Binde-, Gleit-, Schmier-, Formtrenn-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler, Lösungsbeschleuniger, Antischaummittel, Salzbildner, Gelbildner, Verdickungsmittel, Fließregulierungsmittel, Sorptionsmittel, Mittel zur Erzielung eines Depoteffekts oder Mittel, insbesondere Salze, zur Veränderung des osmotischen Drucks, Überzugsmittel oder Antioxidantien etc. enthalten. Sie können auch zwei oder mehrere Verbindungen der Formel I und noch einen oder mehrere andere therapeutisch wirksame Stoffe enthalten.

Derartige andere therapeutisch wirksame Substanzen können beispielsweise sein: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Benzafibrat, Fenofibrat; Mittel für die Thromboseprophylaxe, wie z.B. Phenprocoumon.

In den pharmazeutischen Präparaten kann der Gehalt an dem Wirkstoff oder den Wirkstoffen der Formel I in weiten Grenzen schwanken und z.B. 0,05 bis 50 Gew.%, vorzugsweise 0,05 bis 20 Gew.%, betragen. In festen Darreichungsformen, wie Dragees, Tabletten etc. beträgt der Gehalt an einem oder mehreren Wirkstoffen der Formel I in vielen Fällen 2 bis 20 Gew.%. Flüssige Darreichungsformen, wie Tropfen, Emulsionen und Injektionslösungen enthalten häufig 0,05 bis 2 Gew.%, vorzugsweise 0,05 bis 1 Gew.%, eines oder mehrerer Wirkstoffe der Formel I. Der Gehalt an einem oder mehreren Wirkstoffen der Formel I kann gegebenenfalls in den pharmazeutischen Präparaten teilweise, z.B. bis zu 50 Gew.%, vorzugsweise zu 5 bis 40 Gew.%, durch eine oder mehrere andere therapeutisch wirksame Substanzen ersetzt sein.

Die Verbindungen der Formel I und die pharmazeutischen Präparate, welche die Verbindungen der Formel I als Wirkstoffe enthalten, können am Menschen bei der Bekämpfung bzw. Vorbeugung von Thrombosen und von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis des Wirkstoffs oder Wirkstoffgemisches von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l CaCl₂ löst dann eine Kontraktion aus. Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= IC₅₀, mol/l). In der folgenden Tabelle sind die so erhaltenen IC₅₀-Werte angegeben. Wie der Vergleich mit dem IC₅₀-Wert 3·10⁻⁴ für die bekannte Verbindung Molsidomin (N-Ethoxycarbonyl-3-morpholino-sydnonimin), vgl. DE-B-16 95 897, ergibt, liegen die Werte für die Verbindungen der Formel I erheblich günstiger.

**Tabelle**

| Verbindungen der Formel I gemäß Beispiel | IC₅₀ (mol/l) |
|---|---|
| 1 | 3.10⁻⁶ |
| 4 | 6.10⁻⁵ |
| 5 | 6.10⁻⁷ |
| Molsidomin (N-Ethoxycarbonyl-3-morpholinosydnonimin) Vergleichssubstanz | >1.10⁻⁴ |

### Beispiel 1

### 3-tert-Pentyl-1.2.3.4-oxatriazolium-5-olat

### a) tert-Pentyl-hydrazin

Die Mischung, bestehend aus 68 g Hydrazinhydrat, 161 ml Wasser, 80 g tert-Pentylalkohol und 31 g konz. Salzsäure wird 3 h auf 60°C erwärmt. Nach dieser Zeit wird durch Anbringen einer Destillationsbrücke der flüchtige Anteil bis zum Siedepunkt von 80°C abdestilliert. Nach dem Abkühlen wird der Rückstand mit 100 ml Methylenchlorid gewaschen und mit Pottasche alkalisch gestellt und das Produkt mit Methylenchlorid extrahiert. Die Methylenchloridphase wird über Natriumsulfat getrocknet und eingeengt.
Ausbeute: 18 g Öl

### b) 1-tert-Pentyl-semicarbazid

Zur im Eisbad gekühlten Mischung von 17 g tert-Pentylhydrazin und 30 ml Wasser werden 13,6 g Kaliumcyanat gegeben und anschlielßend 16,5 g konz. Salzsäure zugetropft. Es wird 3 h bei Raumtemperatur gerührt und der Feststoff abgesaugt, mit Wasser gewaschen und im Vakuum getrocknet.
Ausbeute: 12 g Fp.166 bis 168°C

### c) 1-tert-Pentyl-1-nitroso-semicarbazid

1-tert-Pentyl-semicarbazid (12 g) wird in der Mischung aus 50 ml Wasser und 25 ml Methanol im Eisbad abgekühlt und mit 5,6 g Natriumnitrit versetzt. In diese Lösung wird konz. Salzsäure zugetropft, bis ein pH-Wert von 4 bis 5 eingestellt ist.

Die Reaktionsmischung wird über Nacht bei Raumtemperatur gerührt. Der Niederschlag wird abgesaugt und im Vakuum getrocknet.
Ausbeute: 9,8 g Fp. 111 bis 113°C

### d) 3-tert-Pentyl-1.2.3.4-oxatriazolium-5-olat

Die oben beschriebene Nitrosoverbindung (9,8 g) wird in 30 ml Chloroform gelöst. Nach Zufügen von 4 ml Eisessig wird die Lösung 2 h auf 60°C erwärmt. Nach dem Abkühlen auf Raumtemperatur wird dreimal mit je 30 ml Wasser ausgeschüttelt und die Chloroformphase über Natriumsulfat getrocknet und eingedampft. Der ölige Rückstand wird im Vakuum destilliert.
Ausbeute: 5,1 g Öl Kp: 92 bis 95°C (bei einem Druck von 1,06 mbar)

### Beispiel 2

### 3-(1-Methyl-1-morpholinocarbonyl-ethyl)-1.2.3.4-oxatriazolium-5-olat

### a) 1-Hydrazino-isobuttersäure-morpholid-hydrochlorid

Die Lösung von 63 g α-Brom-isobuttersäure-morpholid und 40 g Hydrazinhydrat in 250 ml Ethanol wird 24 h auf 70°C erwärmt. Die flüchtigen Anteile werden im Vakuum abdestilliert, der Rückstand mit 100 ml Wasser und 50 ml konz. Salzsäure versetzt und die nichtbasischen Anteile durch Ausschütteln mit Methylenchlorid abgetrennt. Dann wird die wäßrige Phase mit Natronlauge neutralisiert und mit viel Pottasche versetzt. Das Produkt wird mit Methylenchlorid extrahiert. Nach dem Trocknen und Einengen der organischen Phase verbleibt ein gelbliches Öl.
Ausbeute: 17,9 g Fp.(Hydrochlorid): 208 bis 210°C

### b) 1-(1-Methyl-1-morpholinocarbonyl-ethyl)-semicarbazid

Die Lösung von 10 g des oben erwähnten Öls und von 4,3 g Kaliumcyanat in 30 ml Wasser wird mit konz. Salzsäure neutralisiert und 4 h bei Raumtemperatur gerührt. Die Bildung des neuen Produktes wird dünnschichtchromatographisch (Laufmittel Methylenchlorid : Methanol = 9 : 1; Kieselgel) verfolgt und ist nach dieser Zeit beendet.

### c) 1-Nitroso-1-(1-methyl-1-morpholinocarbonyl-ethyl)-semicarbazid

Zur oben erhaltenen Reaktionsmischung werden 4,3 g Natriumnitrit gegeben, und mit konz. Salzsäure wird ein pH von 2 bis 3 eingestellt, wobei im Eisbald gekühlt wird. Es wird 1 h bei Raumtemperatur gerührt und dann die Nitrosoverbindung mit Essigester ausgeschüttelt. Nach dem Trocknen und Einengen verbleibt ein harziger Rückstand, der ohne weitere Reinigung weiterverarbeitet wird.

### d) 3-(1-Methyl-1-morpholinocarbonyl-ethyl)-1.2.3.4-oxatriazolium-5-olat

Das oben erhaltene harzige Produkt wird in 100 ml 2 N HCl 6 h lang bei Raumtemperatur gerührt. Der Feststoff wird abgesaugt, mit Wasser nachgewaschen und im Vakuum getrocknet.
Ausbeute: 6,2 g Fp. 123 bis 124°C

### Beispiel 3

### 3-(1-Methyl-1-piperidinocarbonyl-ethyl)-1.2.3.4-oxatriazolium-5-olat

Die zur Herstellung der Verbindung benötigten Vorprodukte 3a) bis 3c) werden analog Beispiel 2a) bis 2c) hergestellt.
3c) 1-Nitroso-1-(1-methyl-1-piperidinocarbonyl-ethyl)-semicarbazid Fp. 138 bis 151°C

### 3d) 3-(1-Methyl-1-piperidinocarbonyl-ethyl)-1.2.3.4-oxatriazolium-5-olat

Die Verbindung 3c) (4,5 g) wird in 50 ml Wasser suspendiert. Mit konz. Salzsäure wird ein pH von 2 eingestellt und die Mischung 1 h auf 60°C erwärmt. Nach dem Abkühlen wird der Feststoff abgesaugt und aus Isopropanol umkristallisiert.
Ausbeute: 2,4 g Fp: 109 bis 111°C

### Beispiel 4

### 3-(1-tert-Butylaminocarbonyl-1-methyl-ethyl)-1.2.3.4-oxatriazolium-5-olat

Die zur Herstellung der Verbindung benötigten Vorprodukte 4a) bis 4c) werden analog Beispiel 2a) bis 2c) hergestellt.
4c) 1-Nitroso-1-(1-tert-butylaminocarbonyl-1-methyl-ethyl)-semicarbazid Fp. 165 bis 166°C

### 4d) 3-(1-tert-Butylaminocarbonyl-1-methyl-ethyl)-1.2.3.4-oxatriazolium-5-olat

Das Gemisch aus 12,0 g der Verbindung 4c), 30 ml Eisessig und 70 ml Chloroform wird 3 h unter Rückfluß erhitzt. Nach dem Abkühlen auf Raumtemperatur wird mit Natriumbicarbonatlösung und mit Wasser gewaschen, die Chloroformlösung mit Natriumsulfat getrocknet und eingeengt. Der Rückstand wird aus tert-Butyl-methylether umkristallisiert.
Ausbeute: 4,3 g Fp. 119 bis 121°C

### Beispiel 5

### 3-(2-Methyl-pent-2-yl)-1.2.3.4-oxatriazolium-5-olat

### a) (2-Methyl-pent-2-yl)-hydrazin-hydrochlorid

Zur Lösung von Propylmagnesiumbromid, hergestellt aus 44,3 g Magnesium und 224 g Propylbromid in 350 ml Ether, wird Acetonazin (93 g) unter Rückfluß zugetropft. Nach 3 Tage Erhitzen unter Rückfluß wird die Mischung auf eine eisgekühlte wäßrige Ammoniumchloridlösung (500 ml) gegossen und das Produkt mit Ether extrahiert. Die Etherphase wird eingeengt, und der ölige Rückstand wird in 200 ml Wasser und 100 ml konz. Salzsäure 2 h bei Raumtemperatur gerührt. Die nicht basischen Anteile werden mit tert-Butylmethylether entfernt und die wäßrige Lösung wird am Rotationsverdampfer eingeengt.
Ausbeute: 24,2 g (Rohprodukt)

### b) 1-(2-Methyl-pent-2-yl)-semicarbazid

Das Rohprodukt aus Beispiel 5a) wird in 75 ml Wasser auf 0°C abgekühlt, mit 14 g Kaliumcyanat versetzt und 20 h bei Raumtemperatur gehalten. Diese Mischung wird bei pH 2, 4 und 6 jeweils mit 50 ml Methylenchlorid ausgeschüttelt und die bei pH 4 und 6 anfallenden organischen Phasen werden vereinigt, getrocknet und eingeengt.
Ausbeute: 6 g (Rohprodukt)

### c) 1-(2-Methyl-pent-2-yl)-1-nitroso-semicarbazid

Das Rohprodukt aus Beispiel 5b) wird in 100 ml Wasser und 4 ml konz. Salzsäure gelöst, auf 0 bis 5°C abgekühlt und die entstandene Mischung mit 3,2 g Natriumnitrit versetzt und 3 h bei Raumtemperatur gerührt. Die Nitrosoverbindung wird mit Methylenchlorid extrahiert. Die organische Phase wird getrocknet und eingeengt.
Ausbeute: 5,8 g Öl

### d) 3-Methyl-pent-2-yl)-1.2.3.4-oxatriazolium-5-olat

Die Lösung von 5 g des Öls aus Beispiel 5c) in 50 ml Essigester wird mit 2,4 g Eisessig versetzt und 2 h auf 60°C erwärmt. Nach dem Erkalten wird mit 3 Portionen Wasser zu je 50 ml gewaschen und die Essigesterphase über Natriumsulfat getrocknet und eingeengt. Der ölige Rückstand wird säulenchromatographisch gereinigt (Kieselgel, Laufmittel : Methylenchlorid). Die sauberen Fraktionen werden vereint und eingeengt.
Ausbeute: 3,9 g Fp. 30 bis 32°C

### Beispiel 6

Analog Beispiel 5 läßt sich die Verbindung 3-(3-Methylpent-3-yl)-1.2.3.4-oxatriazolium-5-olat in Form eines Öles herstellen.

### Beispiel 7

Analog Beispiel 5 läßt sich die Verbindung 3-(2.3-Dimethyl-but-2-yl)-1.2.3.4-oxatriazolium-5-olat herstellen.
Fp.: 38°C.

In den nachfolgenden Beispielen werden pharmazeutische Präparate beschrieben:

### Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q.s. |
| Natriumcarboxymethylcellulose | 0,6 g |
| Polyoxyethylenstearat | q.s. |
| Reinglyzerol | 0,2 bis 2 g |
| Aromastoffe | q.s. |
| Wasser (entmineralisiert oder destilliert) ad | 100 ml |

### Beispiel B

Dragees können nach folgenden Rezepturen hergestellt werden:

| | |
|---|---|
| a) Wirkstoff | 46 mg |
| Milchzucker | 90 mg |
| Maisstärke | 90 mg |
| sec Calciumphosphat | 34 mg |
| lösliche Stärke | 3 mg |
| Magnesiumstearat | 3 mg |
| kolloidale Kieselsäure | 4 mg |
| | 2̅7̅0̅ m̅g̅ |

| | |
|---|---|
| b) Wirkstoff | 10 mg |
| Milchzucker | 60 mg |
| Maisstärke | 90 mg |
| sec Calciumphosphat | 30 mg |
| lösliche Stärke | 3 mg |
| Magnesiumstearat | 3 mg |
| kolloidale Kieselsäure | 4 mg |
| | 2̅0̅0̅ m̅g̅ |

| | |
|---|---|
| c) Wirkstoff | 1 mg |
| Maisstärke | 100 mg |
| Milchzucker | 60 mg |
| sec Calciumphosphat | 30 mg |
| lösliche Stärke | 3 mg |
| Magnesiumstearat | 2 mg |
| kolloidale Kieselsäure | 4 mg |
| | 2̅0̅0̅ m̅g̅ |

### Beispiel C

Tabletten können nach folgenden Formulierungen hergestellt werden:

| | |
|---|---|
| a) Wirkstoff | 2 mg |
| Milchzucker | 60 mg |
| Maisstärke | 30 mg |
| lösliche Stärke | 4 mg |
| Magnesiumstearat | 4 mg |
| | 1̅0̅0̅ m̅g̅ |

| | |
|---|---|
| b) Wirkstoff | 24 mg |
| Milchzucker | 56 mg |
| Maisstärke | 30 mg |
| lösliche Stärke | 5 mg |
| Magnesiumstearat | 5 mg |
| | 1̅2̅0̅ m̅g̅ |

### Beispiel D

Für die Herstellung von Weichgelatinekapseln mit 5 mg Wirkstoff pro Kapsel eignet sich die folgende Zusammensetzung

| | |
|---|---|
| Wirkstoff | 5 mg |
| Mischung von Triglyzeriden aus Kokosöl | 150 mg |
| Kapselinhalt | 1̅5̅5̅ m̅g̅ |

### Beispiel E

Für die Herstellung des Inhalts von Hartgelatinekapseln eignen sich die folgenden Rezepturen:

| | |
|---|---|
| a) Wirkstoff | 10 mg |
| Maisstärke | 190 mg |
| | 2̅0̅0̅ m̅g̅ |

| | |
|---|---|
| b) Wirkstoff | 40 mg |
| Milchzucker | 80 mg |
| Maisstärke | 80 mg |
| | 2̅0̅0̅ m̅g̅ |

### Beispiel F

Suppositorien können nach folgender Rezeptur hergestellt werden:

| | |
|---|---|
| Wirkstoff | 20 mg |
| Suppositoriengrundmasse ad | 2 g |

### Beispiel G

Injektionslösungen können nach folgenden Rezepturen hergestellt werden:

| | |
|---|---|
| a) Wirkstoff | 1,0 mg |
| Polyethylenglykol 400 | 0,3 mg |
| Natriumchlorid | 2,7 mg |
| Aqua ad injectabila ad | 1 ml |

| | |
|---|---|
| b) Wirkstoff | 4,0 mg |
| Natriumchlorid | 9,0 mg |
| Aqua ad injectabila ad | 1 ml |

### Beispiel H

Tropfen können nach folgender Rezeptur hergestellt werden (20 mg Wirkstoff in 1 ml = 20 Tropfen):

| | |
|---|---|
| Wirkstoff | 2,00 g |
| Benzoesäuremethylester | 0,07 g |
| Benzoesäureethylester | 0,03 g |
| Ethanol 96%ig | 4 ml |
| entmineralisiertes Wasser ad | 100 ml |

## Patentansprüche (Patentansprüche für folgende(n) Vertragsstaat(en): AT, BE, CH, DE, FR, GB, IT, LI, NL)

1. In 3-Stellung substituierte 1.2.3.4-Oxatriazolium-5-olate der allgemeinen Formel I 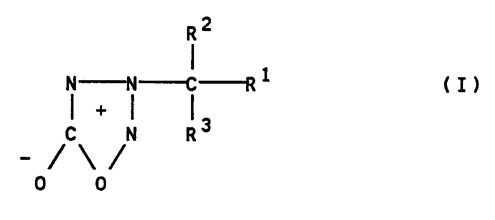 worin
R¹ (C₂ bis C₄)Alkyl oder -CONR⁴R⁵,
R², R³ unabhängig voneinander (C₁ bis C₄)Alkyl,
R⁴ Wasserstoff, (C₁ bis C₄)Alkyl und
R⁵ (C₁ bis C₄)Alkyl bedeuten, oder
R⁴ und R⁵ zusammen mit dem Stickstoffatom, an das sie gebunden sind, einen 5-, 6- oder 7-gliedrigen heterocyclischen Ring bilden, der auch noch ein oder mehrere Heteroatome aus der Reihe N, O oder S enthalten kann.

2. 1.2.3.4-Oxatriazolium-5-olate nach Anspruch 1, dadurch gekennzeichnet, daß R¹ (C₂ bis C₄)Alkyl, vorzugsweise Ethyl, bedeutet.

3. 1.2.3.4-Oxatriazolium-5-olate nach Anspruch 1, dadurch gekennzeichnet, daß R¹ -CONR⁴R⁵ bedeutet und R⁴ und R⁵ zusammen mit dem Stickstorfatom, an das sie gebunden sind, einen Piperidin-, Pyrrolidin-, Morpholin- oder Piperazinrest bedeuten.

4. 1.2.3.4-Oxatriazolium-5-olate nach einem oder mehreren der Ansprüche 1 bis 3, dadurch gekennzeichnet, daß R² und R³ beide Methyl bedeuten.

5. 3-tert-Pentyl-1.2.3.4-oxatriazolium-5-olat.

6. 3-(1-tert-Butylaminocarbonyl-1-methyl-ethyl)-1.2.3.4-oxatriazolium-5-olat.

7. 3-(2-Methyl-pent-2-yl)-1.2.3.4-oxatriazol-5-olat.

8. Verfahren zur Herstellung der 1.2.3.4-Oxatriazolium-5-olate eines oder mehrerer der Ansprüche 1 bis 7, dadurch gekennzeichnet, daß eine Nitrosoverbindung der allgemeinen Formel II 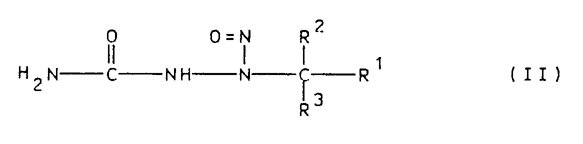 worin R¹, R² und R³ die in Anspruch 1 angegebenen Bedeutungen besitzen, in einem Lösungsmittel durch Zusatz einer Säure bei Temperaturen von 5°C bis zum Siedepunkt des Lösungsmittels, vorzugsweise von Raumtemperatur bis 100°C, ganz besonders bevorzugt von Raumtemperatur bis 80°C, oder in einem Lösungsmittel durch Erhitzen auf Temperaturen von 30°C bis zum Siedepunkt des Lösungsmittels, vorzugsweise auf Temperaturen von 30 bis 100°C, und ganz besonders bevorzugt auf Temperaturen von 50 bis 80°C, cyclisiert wird.

9. Verwendung der 1.2.3.4-Oxatriazolium-5-olate eines oder mehrerer der Ansprüche 1 bis 7 zur Herstellung von pharmazeutischen Präparaten, insbesondere zur Herstellung von pharmazeutischen Präparaten mit antianginöser oder antithrombotischer Wirkung.

10. Pharmazeutisches Präparat, enthaltend einen Wirkstoff und einen pharmazeutisch inerten Trägerstoff sowie gegebenenfalls übliche Hilfs- und Zusatzstoffe, dadurch gekennzeichnet, daß es einen Wirkstoff eines oder mehrerer der Ansprüche 1 bis 7, insbesondere in Mengen von 0,05 bis 25 Gew.%, vorzugsweise in Mengen von 0,05 bis 20 Gew.%, enthält.

## Patentansprüche (Patentansprüche für folgende(n) Vertragsstaat(en): ES)

1. Verfahren zur Herstellung von in 3-Stellung substituierten 1.2.3.4-Oxatriazolium-5-olate der allgemeinen Formel I 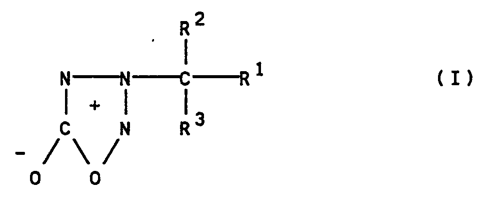 worin
R¹ (C₂ bis C₄)Alkyl oder -CONR⁴R⁵,
R², R³ unabhängig voneinander (C₁ bis C₄)Alkyl,
R⁴ Wasserstoff, (C₁ bis C₄)Alkyl und
R⁵ (C₁ bis C₄)Alkyl bedeuten, oder
R⁴ und R⁵ zusammen mit dem Stickstoffatom, an das sie gebunden sind, einen 5-, 6- oder 7-gliedrigen heterocyclischen Ring bilden, der auch noch ein oder mehrere Heteroatome aus der Reihe N, O oder S enthalten kann,
dadurch gekennzeichnet, daß eine Nitrosoverbindung der allgemeinen Formel II 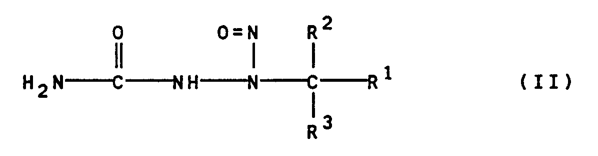 worin R¹, R² und R³ die oben angegebenen Bedeutungen besitzen, in einem Lösungsmittel durch Zusatz einer Säure bei Temperaturen von 5°C bis zum Siedepunkt des Lösungsmittels oder in einem Lösungsmittel durch Erhitzen auf Temperaturen von 30°C bis zum Siedepunkt des Lösungsmittels cyclisiert wird.

2. Verfahren nach Anspruch 1, dadurch gekennzeichnet, daß die Verbindung der Formel II in einem Lösungsmittel durch Zusatz einer Säure bei einer Temperatur von Raumtemperatur bis 100°C, bevorzugt von Raumtemperatur bis 80°C, oder in einem Lösungsmittel durch Erhitzen auf eine Temperatur von 30 bis 100°C, bevorzugt auf eine Temperatur von 50 bis 80°C, cyclisiert wird.

3. Verfahren nach Anspruch 1 und/oder 2, dadurch gekennzeichnet, daß eine Verbindung der Formel II eingesetzt wird, bei der R¹ (C₂ bis C₄) Alkyl, insbesondere Ethyl, bedeutet.

4. Verfahren nach Anspruch 1 und/oder 2, dadurch gekennzeichnet, daß eine Verbindung der Formel II eingesetzt wird, bei de R¹ -CONR⁴R⁵ bedeutet und R⁴ und R⁵ zusammen mit dem Stickstoffatom, an das sie gebunden sind, einen Piperidin-, Pyrrolidin-, Morpholin- oder Piperazinrest bedeuten.

5. Verfahren nach Anspruch 1 und/oder 2, und/oder einem der Ansprüche 3 oder 4, dadurch gekennzeichnet, daß eine Verbindung der Formel II eingesetzt wird, bei der R² und R³ beide Methyl bedeuten.

6. Verfahren nach einem oder mehreren der Ansprüche 1 bis 3 und/oder 5, dadurch gekennzeichnet, daß eine Verbindung der Formel II eingesetzt wird, bei der R¹ Ethyl und R² und R³ beide Methyl bedeuten.

7. Verfahren nach einem oder mehreren der Ansprüche 1 und/oder 2 und/oder 5,dadurch gekennzeichnet, daß eine Verbindung der Formel II eingesetzt wird, bei der R¹ -CONR⁴R⁵ bedeutet und R⁴ Wasserstoff, R⁵ tert-Butyl und R² und R³ beide Methyl bedeuten.

8. Verfahren nach einem oder mehreren der Ansprüche 1 bis 3 und/oder 5, dadurch gekennzeichnet, daß eine Verbindung der Formel II eingesetzt wird, bei der R¹ n-Propyl und R² und R³ beide Methyl bedeuten.

9. Verwendung der nach einem oder mehreren der Ansprüche 1 bis 8 erhältlichen 1.2.3.4-Oxatriazolium-5-olate zur Herstellung von pharmazeutischen Präparaten, insbesondere zur Herstellung von pharmazeutischen Präparaten mit antianginöser oder antithrombotischer Wirkung.

10. Verwendung nach Anspruch 9 zur Herstellung pharmazeutischer Präparate, die 0.05 bis 25 Gew.%, vorzugsweise 0.05 bis 20 Gew.% des Wirkstoffs enthalten.

## Claims (Claims for the following Contracting State(s): AT, BE, CH, DE, FR, GB, IT, LI, NL)

1. 1,2,3,4-Oxatriazolium-5-olates substituted in the 3-position, of the general formula I 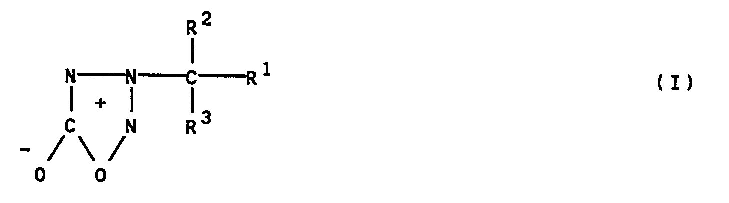 in which
R¹ denotes (C₂ to C₄)alkyl or -CONR⁴R⁵,
R² and R³, independently of one another, denote (C₁ to C₄)alkyl,
R⁴ denotes hydrogen or (C₁ to C₄)alkyl, and
R⁵ denotes (C₁ to C₄)alkyl or
R⁴ and R⁵, together with the nitrogen atom to which they are bonded, denote a five-membered, six-membered or seven-membered heterocyclic ring which may also contain one or more hetero atoms selected from the series comprising N, O or S.

2. 1,2,3,4-Oxatriazolium-5-olates according to Claim 1, characterized in that R¹ denotes (C₂ to C₄)alkyl, preferably ethyl.

3. 1,2,3,4-Oxatriazolium-5-olates according to Claim 1, characterized in that R¹ denotes -CONR⁴R⁵ and R⁴ and R⁵, together with the nitrogen atom to which they are bonded, denote a piperidine, pyrrolidine, morpholine or piperazine radical.

4. 1,2,3,4-Oxatriazolium-5-olates according to one or more of Claims 1 to 3, characterized in that R² and R³ both denote methyl.

5. 3-tert-Pentyl-1,2,3,4-oxatriazolium-5-olate.

6. 3-(1-tert-Butylaminocarbonyl-1-methyl-ethyl)-1,2,3,4-oxatriazolium-5-olate.

7. 3-(2-Methyl-pent-2-yl)-1,2,3,4-oxatriazolium-5-olate.

8. Process for the preparation of the 1,2,3,4-oxatriazolium-5-olates of one or more of Claims 1 to 7, characterized in that a nitroso compound of the general formula II 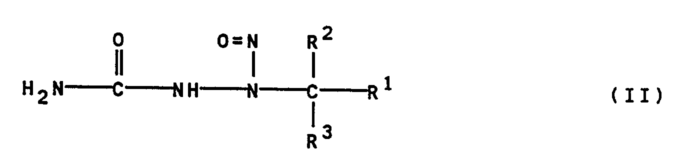 in which R¹, R² and R³ have the meanings given in Claim 1, is cyclized in a solvent by addition of an acid at temperatures from 5°C up to the boiling point of the solvent, preferably from room temperature up to 100°C, very particularly preferably from room temperature up to 80°C, or in a solvent by heating at temperatures of 30°C up to the boiling point of the solvent, preferably at temperatures of 30 to 100°C, and very particularly preferably at temperatures of 50 to 80°C.

9. Use of the 1,2,3,4-oxatriazolium-5-olates of one or more of Claims 1 to 7, for the production of pharmaceutical preparations, in particular for the production of pharmaceutical preparations having an antianginal or antithrombotic effect.

10. Pharmaceutical preparation containing an active ingredient and a pharmaceutically inert carrier as well as, optionally, customary auxiliaries and additives, characterized in that it contains an active ingredient of one or more of Claims 1 to 7, particularly in amounts of 0.05 to 25 % by weight, preferably in amounts of 0.05 to 20 % by weight.

## Claims (Claims for the following Contracting State(s): ES)

1. Process for preparing 1,2,3,4-oxatriazolium-5-olates substituted in the 3-position, of the general formula I 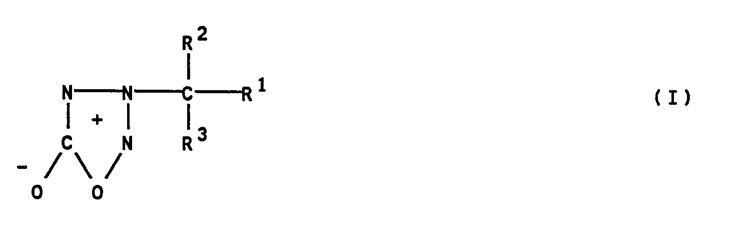 in which
R¹ denotes (C₂ to C₄)alkyl or -CONR⁴R⁵,
R² and R³, independently of one another, denote (C₁ to C₄)alkyl,
R⁴ denotes hydrogen or (C₁ to C₄)alkyl, and
R⁵ denotes (C₁ to C₄)alkyl or
R⁴ and R⁵, together with the nitrogen atom to which they are bonded, denote a five-membered, six-membered or seven-membered heterocyclic ring which may also contain one or more hetero atoms selected from the series comprising N, O or S,
characterized in that a nitroso compound of the general formula II 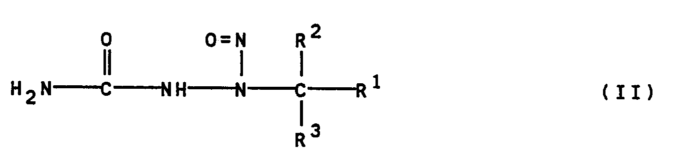 in which R¹, R² and R³ have the meanings given above, is cyclized in a solvent by addition of an acid at temperatures from 5°C up to the boiling point of the solvent or in a solvent by heating at temperatures of 30°C up to the boiling point of the solvent.

2. Process according to Claim 1, characterized in that the compound of formula II is cyclized in a solvent by addition of an acid at temperatures from room temperature up to 100°C, preferably from room temperature up to 80°C, or in a solvent by heating at temperatures of 30 to 100°C, preferably at temperatures of 50 to 80°C.

3. Process according to Claim 1 and/or 2, characterized in that a compound of the formula II is employed wherein R¹ denotes (C₂ to C₄)alkyl, preferably ethyl.

4. Process according to Claim 1 and/or 2, characterized in that a compound of the formula II is employed wherein R¹ denotes -CONR⁴R⁵ and R⁴ and R⁵, together with the nitrogen atom to which they are bonded, denote a piperidine, pyrrolidine, morpholine or piperazine radical.

5. Process according to Claim 1 and/or 2, and/or one of Claims 3 or 4, characterized in that a compound of the formula II is employed wherein R² and R³ both denote methyl.

6. Process according to one or more of Claims 1 to 3 and/or 5, characterized in that a compound of the formula II is employed wherein R¹ denotes ethyl and R² and R³ both denote methyl.

7. Process according to one or more of Claims 1 and/or 2 and/or 5, characterized in that a compound of the formula II is employed wherein R¹ denotes - CONR⁴R⁵ and R⁴ denotes hydrogen, R⁵ denotes tert.-butyl and R² and R³ both denote methyl.

8. Process according to one or more of Claims 1 to 3 and/or 5, characterized in that a compound of the formula II is employed wherein R¹ denotes n-propyl and R² and R³ both denote methyl.

9. Use of the 1,2,3,4-oxatriazolium-5-olates obtainable according to one or more of Claims 1 to 8, for the production of pharmaceutical preparations, in particular for the production of pharmaceutical preparations having an antianginal or antithrombotic effect.

10. Use according to Claim 9 for preparing pharmaceutical preparations containing 0.05 to 25% by weight, preferably 0.05 to 20% by weight, of active ingredient.

## Revendications (Revendications pour l'(les) Etat(s) contractant(s) suivant(s): AT, BE, CH, DE, FR, GB, IT, LI, NL)

1. 1,2,3,4-oxatriazolium-5-olates substitués en position 3 de formule générale I 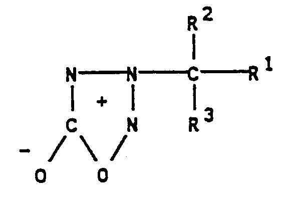 dans laquelle :
R¹ représente un alkyle en C₂ à C₄ ou -CONR⁴R⁵,
R², R³, indépendamment l'un de l'autre, représentent un alkyle en C₁ à C₄,
R⁴ représente les hydrogène, alkyle en C₁ à C₄, et
R⁵ représente un alkyle en C₁ à C₄, ou
R⁴ et R⁵, ensemble avec l'atome d'azote auquel ils sont liés, forment un cycle hétérocyclique à 5, 6 ou 7 chaînons, qui peut encore comporter un ou plusieurs hétéro-atomes de la série des N, O ou S.

2. 1,2,3,4-oxatriazolium-5-olates selon la revendication 1, caractérisés en ce que R¹ signifie un alkyle en (C₂ à C₄), de préférence l'éthyle.

3. 1,2,3,4-oxatriazolium-5-olates selon la revendication 1, caractérisés en ce que R¹ signifie -CONR⁴R⁵, et R⁴ et R⁵, ensemble avec l'atome d'azote auquel ils sont liés, signifient un radical pipéridine, pyrrolidine, morpholine ou pipérazine.

4. 1,2,3,4-oxatriazolium-5-olates selon une ou plusieurs des revendications 1 à 3, caractérisés en ce que R² et R³ signifient, les deux radicaux, un méthyle.

5. 3-tert-pentyl-1,2,3,4-oxatriazolium-5-olate.

6. 3-(1-tert-butylaminocarbonyl-1-méthyl-éthyl)-1,2,3,4-oxatriazolium-5-olate.

7. 3-(2-méthyl-pent-2-yl)-1,2,3,4-oxatriazolium-5-olate.

8. Procédé pour la préparation de 1,2,3,4-oxotraizolium-5-olates selon une ou plusieurs des revendications 1 à 7, caractérisé en ce qu'on cyclise un composé nitroso de formule générale II 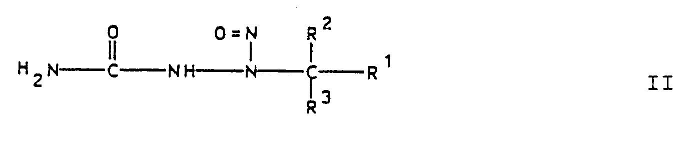 dans laquelle R¹, R² et R³ ont les significations données dans la revendication 1, dans un solvant par addition d'un acide à des températures allant de 5°C jusqu'au point d'ébullition du solvant, de préférence de la température amoiante à 100°C, de manière particulièrement préférée de la température ambiante à 80°C, ou dans un solvant, par chauffage à des températures allant de 30°C jusqu'au point d'ébullition du solvant, de préférence des températures de 30°C à 100°C, et de manière particulièrement préférée à des températures de 50 à 80°C.

9. Utilisation des 1,2,3,4-oxatriazolium-5-olates d'une ou plusieurs des revendications 1 à 7, pour la préparation de préparations pharmaceutiques, plus particulièrement pour la préparation de préparations pharmaceutiques ayant un effet anti-angineux ou anti-thrombique.

10. Préparation pharmaceutique, contenant un principe actif et un agent véhicule pharmaceutiquement inerte ainsi qu'éventuellement des adjuvants et des agents auxiliaires usuels, caractérisée en ce qu'elle contient un principe actif d'une ou de plusieurs des revendications 1 à 7, plus particulièrement en quantités de 0,05 à 25% en poids, de préférence en quantités de 0,05 à 20% en poids.

## Revendications (Revendications pour l'(les) Etat(s) contractant(s) suivant(s): ES)

1. Procédé pour la préparation des 1,2,3,4-oxatriazolium-5-olates substitués en position 3 de formule générale I 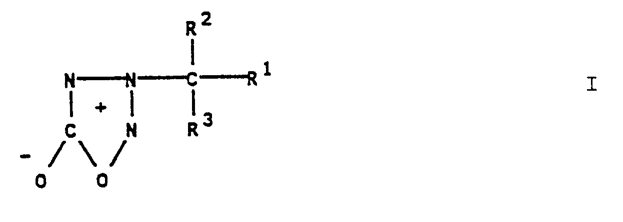 dans laquelle :
R¹ représente un alkyle en C₂ à C₄ ou -CONR⁴R⁵,
R², R³, indépendamment l'un de l'autre, représentent un alkyle en C₁ à C₄,
R⁴ représente les hydrogène, alkyle en C₁ à C₄, et
R⁵ représente un alkyle en C₁ à C₄, ou
R⁴ et R⁵, ensemble avec l'atome d'azote auquel ils sont liés, forment un cycle hétérocyclique à 5, 6 ou 7 chaînons, qui peut comporter encore un ou plusieurs hétéro-atomes de la série des N, O ou S, caractérisé en ce qu'on cyclise un composé nitroso de formule générale II 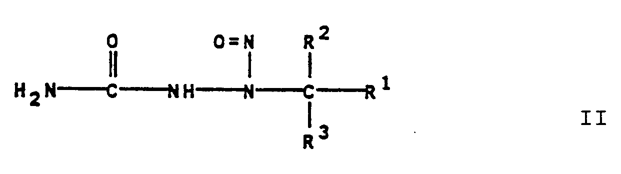
dans laquelle R¹, R² et R³ ont les significations données ci-dessus, dans un solvant, par addition d'un acide à des températures allant de 5°C jusqu'au point d'ébullition du solvant, ou dans un solvant par chauffage à des températures allant de 30°C jusqu'au point d'ébullition du solvant.

2. Procédé selon la revendication 1, caractérisé en ce qu'on cyclise le composé de formule II dans un solvant par addition d'un acide à des températures allant de la température ambiante à 100°C, de préférence de la température ambiante à 80°C, ou dans un solvant par chauffage à une température allant de 30°C à 100°C, de préférence à une température allant de 50 à 80°C.

3. Procédé selon la revendication 1 et/ou 2, caractérisé en ce qu'on utilise un composé de formule II, dans lequel R¹ signifie un alkyle en C₂ à C₄, plus particulièrement l'éthyle.

4. Procédé selon la revendication 1 et/ou 2, caractérisé en ce qu'on utilise un composé de formule II, dans lequel R¹ signifie -CONR⁴R⁵, R⁴ et R⁵, ensemble avec l'atome d'azote auquel ils sont liés, signifient un radical pipéridine, pyrrolidine, morpholine ou pipérazine.

5. Procédé selon la revendication 1 et/ou 2, et/ou une des revendications 3 ou 4, caractérisé en ce qu'on utilise un composé de formule II, dans lequel R² et R³ signifient, les deux, le méthyle.

6. Procédé selon une ou plusieurs des revendications 1 à 3, et/ou 5, caractérisé en ce qu'on utilise un composé de formule II, dans lequel R¹ signifie l'éthyle et R² et R³ signifient, les deux radicaux, le méthyle.

7. Procédé selon une ou plusieurs des revendications 1 et/ou 2 et/ou 5, caractérisé en ce qu'on utilise un composé de formule II, dans lequel R¹ signifie -CONR⁴R⁵ et R⁴ signifie l'hydrogène, R⁵ le tert-butyle et R² et R³, signifient, les deux radicaux, le méthyle.

8. Procédé selon une ou plusieurs des revendications 1 à 3 et/ou 5, caractérisé en ce qu'on utilise un composé de formule II, dans lequel R¹ représente le n-propyle et R² et R³, les deux radicaux, le méthyle.

9. Utilisation des 1,2,3,4-oxatriazolium-5-olates que l'on peut obtenir selon une ou plusieurs des revendications 1 à 8, pour la préparation de préparations pharmaceutiques, plus particulièrement pour la préparation de préparations pharmaceutiques ayant une action anti-angineuse ou anti-thrombique.

10. Utilisation selon la revendication 9, pour la préparation de préparations pharmaceutiques contenant de 0,05 à 25% en poids, de préférence de 0,05 à 20% en poids, de principe actif.
